# EUROPEAN PATENT SPECIFICATION

(11) **EP 3 697 315 B1**
(45) Date of publication and mention of the grant of the patent: **05.01.2022**
(21) Application number: 18793190.2
(22) Date of filing: 17.10.2018
(51) Int. Cl.: A61B 8/12, A61B 8/08, A61B 8/00

(54) **DIGITAL ROTATIONAL PATIENT INTERFACE MODULE**
DIGITALES ROTATIONSPATIENTENSCHNITTSTELLENMODUL
MODULE D'INTERFACE PATIENT ROTATIF NUMÉRIQUE

(30) Priority: 19.10.2017 US 201762574455 P
(43) Date of publication of application: 26.08.2020
(73) Proprietor: Koninklijke Philips N.V., 5656 AG Eindhoven (NL)
(72) Inventor: PEREZ, Cesar, 5656 AE Eindhoven (NL); KANTOR, Sherwood, 5656 AE Eindhoven (NL); DORANDO, Dale, Gene, 5656 AE Eindhoven (NL); ZIEGENBEIN, Randall, 5656 AE Eindhoven (NL)
(74) Representative: Philips Intellectual Property & Standards
(86) International application number: PCT/EP2018/078377
(87) International publication number: WO 2019/076968

(56) References cited:
- CN-U- 205 433 742
- US-A1- 2010 234 736
- US-A1- 2014 187 925
- US-A1- 2014 187 965
- US-A1- 2016 074 005

## Description

### TECHNICAL FIELD

The present disclosure relates generally to intraluminal imaging and, in particular, to receiving and converting imaging signals with a patient interface module (PIM). The PIM is configured to transmit ultrasound echo signals along a differential signal route. The PIM may also be configured to support Ethernet communications.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy with frequencies higher than 2 MHz to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

IVUS catheters may include rotational devices. For a typical rotational IVUS catheter, an ultrasound transducer element is located at the tip of a flexible driveshaft that spins inside a plastic sheath inserted into the vessel of interest. The transducer element is oriented such that the ultrasound beam propagates into the tissue and back. The transducer then listens for the returning echoes reflected from various tissue structures. The returning echoes are generally transmitted to an IVUS processing system along a single analog channel. These transmissions may be prone to electrical noise and electromagnetic coupled interference that may degrade the quality of IVUS images associated with the transmissions. Furthermore, existing IVUS systems typically require an expensive and complex custom cable to transmit signals between the ultrasound transducer element and the processing system. Thus, needs exist for improvements in IVUS imaging systems.

CN 205433742U relates to an intravascular ultrasound system, comprising: a main box, a catheter interface box, an ultrasonic transducer, a display and an input device. The display and the input device are respectively connected to the main box, and the main box is connected with the catheter interface box. The interface box is coupled to the ultrasound transducer; wherein the ADC acquisition module and the analog front end are located in the catheter interface box. The main box and catheter interface box are connected by a transmission cable.

### SUMMARY

A system defined by claim 1 and a method defined by claim 9 for intraluminal ultrasound imaging are provided. An intraluminal ultrasound imaging system may include a patient interface module (PIM) in communication with an intraluminal device positioned within a body lumen of a patient. The PIM may receive ultrasound echo signals, transmit the ultrasound echo signals along a differential signal path, and digitize the ultrasound echo signals. The ultrasound echo signals may also be configured to be transmitted via an Ethernet connection to a processing system connected to the PIM.

Embodiments of the present disclosure provide an intraluminal ultrasound imaging system, which may include: a patient interface module (PIM) communicatively disposed between a processing system and an intraluminal ultrasound device configured to be positioned within a body lumen of a patient, the PIM comprising a transmitter, an analog to digital converter (ADC), and a communication device comprising a communication cable, wherein the PIM is configured to: transmit, with the transmitter, a first signal to the intraluminal ultrasound device; receive an ultrasound echo signal associated with the first signal from the intraluminal ultrasound device; digitize the ultrasound echo signal with the ADC; convert the digitized ultrasound echo signal to a second signal communicable via the communication device; and transmit the second signal to the processing system via the communication cable of the communication device.

In some embodiments, the processing system is configured to generate an intraluminal ultrasound image representative of the ultrasound echo signal based on the second signal and to display the intraluminal ultrasound image on a display device in communication with the processing system. The communication cable may be an Ethernet cable or a USB cable. The PIM may further include a controller in communication with the transmitter, the ADC, and the communication device. The controller may be a field-programmable gate array (FPGA).

In some embodiments, the intraluminal ultrasound device includes: a rotatable, flexible elongate drive cable comprising a proximal portion and a distal portion; and an ultrasound element disposed at the distal portion of the drive cable and configured to obtain imaging data of the body lumen while rotating. The intraluminal ultrasound device may be an intravascular ultrasound (IVUS) device configured to be positioned within a blood vessel. The ultrasound echo signal may travel on a differential signal path to the ADC within the PIM. In some embodiments, the differential signal path comprises one or more amplifiers and bandpass filters.

A method of intraluminal ultrasound imaging is also provided, which may include: transmitting a first signal with an intraluminal ultrasound device positioned within a body lumen of a patient; receiving, with a patient interface module (PIM) communicatively disposed between the intraluminal ultrasound device and a processing system, an ultrasound echo signal associated with the first signal from the intraluminal ultrasound device; digitizing the ultrasound echo signal with an ADC in the PIM; converting, with a controller in the PIM, the digitized ultrasound echo signal to a second signal communicable via a communication device; and transmitting the second signal to the processing system via the communication device.

The method may also include displaying, with a display device in communication with the processing system, an intraluminal ultrasound image representative of the ultrasound echo signal. The method may include formatting, by the PIM, the ultrasound echo signal according to an image display format of the display device. The communication device may be an Ethernet cable or a USB cable. The controller may be a field-programmable gate array (FPGA).

In some embodiments, the method further includes converting the digitized ultrasound echo signal to a second signal with an Ethernet physical layer (PHY) device. The method may include transmitting the ultrasound echo signals along a differential signal path to the ADC within the PIM. The intraluminal ultrasound device may include: a rotatable, flexible elongate drive cable comprising a proximal portion and a distal portion; and an ultrasound element disposed at the distal portion of the drive cable and configured to obtain imaging data of the body lumen while rotating. The intraluminal ultrasound device may be an intravascular ultrasound (IVUS) device configured to be positioned within a blood vessel.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is diagrammatic schematic view of an intraluminal ultrasound imaging system according to some embodiments of the present disclosure.
Fig. 2 is diagrammatic schematic view of an intraluminal ultrasound imaging system according to some embodiments of the present disclosure.
Fig. 3 is diagrammatic schematic view of a rotational ultrasound device according to some embodiments of the present disclosure.
Fig. 4 is diagrammatic view of a rotational ultrasound device in situ within anatomy of a patient according to some embodiments of the present disclosure.
Fig. 5 is diagrammatic perspective view of a patient interface module (PIM) according to some embodiments of the present disclosure.
Fig. 6 is a flow diagram of an ultrasound imaging method according to embodiments of the present disclosure.

### DETAILED DESCRIPTION

For the purposes of promoting an understanding of the principles of the present disclosure, reference will now be made to the embodiments illustrated in the drawings, and specific language will be used to describe the same.

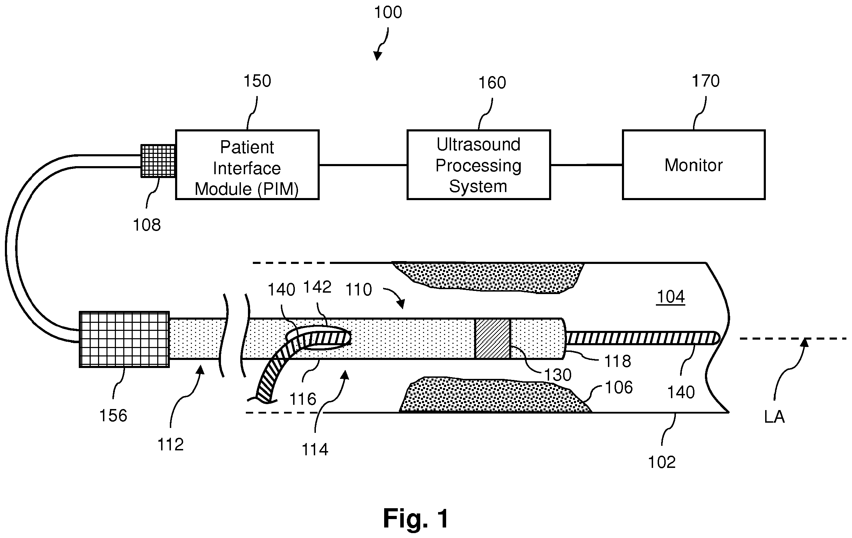

Fig. 1 is a diagrammatic schematic view of an ultrasound system 100 according to some embodiments of the present disclosure. The ultrasound system 100 may be used to carry out intravascular ultrasound imaging of a lumen of a patient. The system 100 may include an ultrasound device 110, a patient interface module (PIM) 150, an ultrasound processing system 160, and/or a monitor 170. The ultrasound device 110 is structurally arranged (*e*.*g*., sized and/or shaped) to be positioned within anatomy 102 of a patient. The ultrasound device 110 obtains ultrasound imaging data from within the anatomy 102. The ultrasound processing system 160 can control the acquisition of ultrasound imaging and may be used to generate an image of the anatomy 102 (using the ultrasound imaging data received via the PIM 150) that is displayed on the monitor 170.

In some embodiments, the system 100 and/or the PIM 150 can include features similar to those described in U.S. Patent Application No. 62/574655, titled "WIRELESS DIGITAL PATIENT INTERFACE MODULE USING WIRELESS CHARGING," filed Oct. 19, 2017, U.S. Patent Application No. 62/574687, titled "INTRALUMINAL DEVICE REUSE PREVENTION WITH PATIENT INTERFACE MODULE AND ASSOCIATED DEVICES, SYSTEMS, AND METHODS," filed Oct. 19, 2017, U.S. Patent Application No. 62/574835, titled "INTRALUMINAL MEDICAL SYSTEM WITH OVERLOADED CONNECTORS," filed Oct. 20, 2017, and U.S. Patent Application No. 62/574610, titled "HANDHELD MEDICAL INTERFACE FOR INTRALUMINAL DEVICE AND ASSOCIATED DEVICES, SYSTEMS, AND METHODS," filed Oct. 19, 2017.

Generally, the ultrasound device 110 can be a catheter, a guide catheter, or a guide wire. The ultrasound device 110 includes a flexible elongate member 116. As used herein, "elongate member" or "flexible elongate member" includes at least any thin, long, flexible structure structurally arranged (e.g., sized and/or shaped) to be positioned within a lumen 104 of the anatomy 102. For example, a distal portion 114 of the flexible elongate member 116 is positioned within the lumen 104, while a proximal portion 112 of the flexible elongate member 116 is positioned outside of the body of the patient. The flexible elongate member 116 can include a longitudinal axis LA. In some instances, the longitudinal axis LA can be a central longitudinal axis of the flexible elongate member 116. In some embodiments, the flexible elongate member 116 can include one or more polymer/plastic layers formed of various grades of nylon, Pebax, polymer composites, polyimides, and/or Teflon. In some embodiments, the flexible elongate member 116 can include one or more layers of braided metallic and/or polymer strands. The braided layer(s) can be tightly or loosely braided in any suitable configuration, including any suitable per in count (pic). In some embodiments, the flexible elongate member 116 can include one or more metallic and/or polymer coils. All or a portion of the flexible elongate member 116 may have any suitable geometric cross-sectional profile (*e*.*g*., circular, oval, rectangular, square, elliptical, etc.) or non-geometric cross-sectional profile. For example, the flexible elongate member 116 can have a generally cylindrical profile with a circular cross-sectional profile that defines an outer diameter of the flexible elongate member 116. For example, the outer diameter of the flexible elongate member 116 can be any suitable value for positioning within the anatomy 102, including between approximately 1 Fr (0.33 mm) and approximately 15 Fr (5 mm), including values such as 3.5 Fr (1.16 mm), 5 Fr (1.67 mm), 7 Fr (2.33 mm), 8.2 Fr (2.73 mm), 9 Fr (3 mm), and/or other suitable values both larger and smaller.

The ultrasound device 110 may or may not include one or more lumens extending along all or a portion of the length of the flexible elongate member 116. The lumen of the ultrasound device 110 can be structurally arranged (*e*.*g*., sized and/or shaped) to receive and/or guide one or more other diagnostic and/or therapeutic instruments. If the ultrasound device 110 includes lumen(s), the lumen(s) may be centered or offset with respect to the cross-sectional profile of the device 110. In the illustrated embodiment, the ultrasound device 110 is a catheter and includes a lumen at the distal portion 114 of the flexible elongate member 116. A guide wire 140 extends through the lumen of the ultrasound device 110 between an entry/exit port 142 and an exit/entry port at a distal end 118 of the flexible elongate member 116. Generally, the guide wire 140 is a thin, long, flexible structure that is structurally arranged (*e*.*g*., sized and/or shaped) to be disposed within the lumen 104 of the anatomy 102. During a diagnostic and/or therapeutic procedure, a medical professional typically first inserts the guide wire 140 into the lumen 104 of the anatomy 102 and moves the guide wire 140 to a desired location within the anatomy 102, such as adjacent to an occlusion 106. The guide wire 140 facilitates introduction and positioning of one or more other diagnostic and/or therapeutic instruments, including the ultrasound device 110, at the desired location within the anatomy 102. For example, the ultrasound device 110 moves through the lumen 104 of the anatomy 102 along the guide wire 140. In some embodiments, the lumen of the ultrasound device 110 can extend along the entire length of the flexible elongate member 116. In the illustrated embodiment, the exit/entry port 142 is positioned proximally of components 130 of the ultrasound device 110. In some embodiments, the exit/entry port 142, the exit/entry port at the distal end 118, and/or the lumen of the ultrasound device 110 is positioned distally of the components 130. In some embodiments, the ultrasound device 110 is not used with a guide wire, and the exit/entry port 142 can be omitted from the ultrasound device 110.

The anatomy 102 may represent any fluid-filled or surrounded structures, both natural and man-made. For example, the anatomy 102 can be within the body of a patient. Fluid can flow through the lumen 104 of the anatomy 102. In some instances, the ultrasound device 110 can be referenced as an intraluminal device. The anatomy 102 can be a vessel, such as a blood vessel, in which blood flows through the lumen 104. In some instances, the ultrasound device 110 can be referenced as an intravascular device. In various embodiments, the blood vessel is an artery or a vein of a patient's vascular system, including cardiac vasculature, peripheral vasculature, neural vasculature, renal vasculature, and/or any other suitable anatomy/lumen inside the body. The anatomy 102 can be tortuous in some instances. For example, the device 110 may be used to examine any number of anatomical locations and tissue types, including without limitation, organs including the liver, heart, kidneys, gall bladder, pancreas, lungs, esophagus; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 110 may be used to examine man-made structures such as, but without limitation, heart valves, stents, shunts, filters and other devices.

The occlusion 106 of the anatomy 102 is generally representative of any blockage or other structural arrangement that results in a restriction to the flow of fluid through the lumen 104, for example, in a manner that is deleterious to the health of the patient. For example, the occlusion 106 narrows the lumen 104 such that the cross-sectional area of the lumen 104 and/or the available space for fluid to flow through the lumen 104 is decreased. Where the anatomy 102 is a blood vessel, the occlusion 106 may be a result of plaque buildup, including without limitation plaque components such as fibrous, fibro-lipidic (fibro fatty), necrotic core, calcified (dense calcium), blood, fresh thrombus, and/or mature thrombus. In some instances, the occlusion 106 can be referenced as thrombus, a stenosis, and/or a lesion. Generally, the composition of the occlusion 106 will depend on the type of anatomy being evaluated. Healthier portions of the anatomy 102 may have a uniform or symmetrical profile (*e*.*g*., a cylindrical profile with a circular cross-sectional profile). The occlusion 106 may not have a uniform or symmetrical profile. Accordingly, diseased portions of the anatomy 102, with the occlusion 106, will have a non-symmetric and/or otherwise irregular profile. While the anatomy 102 is illustrated in Fig. 1 as having a single occlusion 106, it is understood that the devices, systems, and methods described herein have similar application for anatomy having multiple occlusions.

The ultrasound device 110 may include ultrasound imaging components 130 disposed at the distal portion 114 of the flexible elongate member 116. The ultrasound imaging components 130 may be configured to emit ultrasonic energy into the anatomy 102 while the device 110 is positioned within the lumen 104. In some embodiments, the components 130 may include various numbers and configurations. For example, some of the components 130 may be configured to transmit ultrasound pulses while other others may be configured to receive ultrasound echoes. The components 130 may be configured to emit different frequencies of ultrasonic energy into the anatomy 102 depending on the type of tissue being imaged and the type of imaging being used.

In some embodiments, the components 130 include ultrasound transducer(s). For example, the components 130 can be configured to generate and emit ultrasound energy into the anatomy 102 in response to being activated by an electrical signal. In some embodiments, the components 130 include a single ultrasound transducer. In some embodiments, the components 130 include an ultrasound transducer array including more than one ultrasound transducer. For example, an ultrasound transducer array can include any suitable number of individual transducers between 2 transducers and 1000 transducers, including values such as 2 transducers, 4 transducers, 36 transducers, 64 transducers, 128 transducers, 500 transducers, 812 transducers, and/or other values both larger and smaller. The ultrasound transducer array including components 130 can be any suitable configuration, such as phased array including a planar array, a curved array, a circumferential array, an annular array, etc. For example, the ultrasound transducer array including components 130 can be a one-dimensional array or a two-dimensional array in some instances.

In some instances, ultrasound imaging components 130 may be part of a rotational ultrasound device. The active area of the ultrasound imaging components 130 can include one or more transducer materials and/or one or more segments of ultrasound elements (*e*.*g*., one or more rows, one or more columns, and/or one or more orientations) that can be uniformly or independently controlled and activated. The active area of the components 130 can be patterned or structured in various basic or complex geometries. The components 130 can be disposed in a side-looking orientation (*e*.*g*., ultrasonic energy emitted perpendicular and/or orthogonal to the longitudinal axis LA) and/or a forward-looking looking orientation (*e*.*g*., ultrasonic energy emitted parallel to and/or along the longitudinal axis LA). In some instances, the components 130 are structurally arranged to emit and/or receive ultrasonic energy at an oblique angle relative to the longitudinal axis LA, in a proximal or distal direction. In some embodiments, ultrasonic energy emission can be electronically steered by selective triggering of ultrasound imaging components 130 in an array.

The ultrasound transducer(s) of the components 130 can be a piezoelectric micromachined ultrasound transducer (PMUT), capacitive micromachined ultrasonic transducer (CMUT), single crystal, lead zirconate titanate (PZT), PZT composite, other suitable transducer type, and/or combinations thereof. Depending on the transducer material, the manufacturing process for ultrasound transducer(s) can include dicing, kerfing, grinding, sputtering, wafer technologies (*e*.*g*., SMA, sacrificial layer deposition), other suitable processes, and/or combinations thereof.

In some embodiments, the components 130 are configured to obtain ultrasound imaging data associated with the anatomy 102, such as the occlusion 106. The ultrasound imaging data obtained by the ultrasound imaging components 130 can be used by a medical professional to diagnose the patient, including evaluating the occlusion 106 of the anatomy 102. For imaging, the components 130 can be configured to both emit ultrasonic energy into the lumen 104 and/or the anatomy 102, and to receive reflected ultrasound echoes representative of fluid and/or tissue of lumen 104 and/or the anatomy 102. As described herein, components 130 can include an ultrasound imaging element, such as an ultrasound transducer and/or an ultrasound transducer array. For example, the components 130 generate and emit ultrasound energy into the anatomy 102 in response to transmission of an electrical signal to the components 130. For imaging, the components 130 may generate and transmit an electrical signal representative of the received reflected ultrasound echoes from the anatomy 102 (*e*.*g*., to the PIM 150 and/or processing system 160). In various embodiments, the ultrasound imaging component 130 can obtain imaging data associated with intravascular ultrasound (IVUS) imaging, forward looking intravascular ultrasound (FL-IVUS) imaging, intravascular photoacoustic (IVPA) imaging, intracardiac echocardiography (ICE), transesophageal echocardiography (TEE), and/or other suitable imaging modalities. In some embodiments, the device 110 can include an imaging component of any suitable imaging modality, such as optical imaging, optical coherence tomography (OCT), etc. In some embodiments, the device 110 can include any suitable sensing component, including a pressure sensor, a flow sensor, a temperature sensor, an optical fiber, a reflector, a mirror, a prism, an ablation element, a radio frequency (RF) electrode, a conductor, and/or combinations thereof. The imaging and/or sensing components can be implemented in the device 110 in lieu of or in addition to the ultrasound component 130.

For diagnosis and/or imaging, the center frequency of the components 130 can be between 2 MHz and 75 MHz, for example, including values such as 2 MHz, 5 MHz, 10 MHz, 20 MHz, 40 MHz, 45 MHz, 60 MHz, 70 MHz, 75 MHz, and/or other suitable values both larger and smaller. For example, lower frequencies (*e*.*g*., between 2 MHz and 10 MHz) can advantageously penetrate further into the anatomy 102, such that more of the anatomy 102 is visible in the ultrasound images. Higher frequencies (*e*.*g*., 50 MHz, 75 MHz) can be better suited to generate more detailed ultrasound images of the anatomy 102 and/or fluid within the lumen 104. In some embodiments, the frequency of the ultrasound imaging components 130 is tunable. For imaging, in some instances, the components 130 can be tuned to receive wavelengths associated with the center frequency and/or one or more harmonics of the center frequency. In some instances, the frequency of the emitted ultrasonic energy can be modified by the voltage of the applied electrical signal and/or the application of a biasing voltage to the ultrasound imaging components 130.

In some embodiments, the ultrasound imaging components 130 are positioned at the distal portion of the flexible elongate member 116. The ultrasound imaging components 130 can include one or more electrical conductors extending along the length from the flexible elongate member 116. The electrical conductor(s) are in communication with the ultrasound imaging components 130 at the distal portion 114, and an interface 156 at the proximal portion 112. The electrical conductors carry electrical signals between the ultrasound processing system 160 and the ultrasound imaging components 130. For example, activation and/or control signals can be transmitted from the processing system 160 to the ultrasound imaging components 130 via the electrical conductors. Electrical signals representative of the reflected ultrasound echoes can be transmitted from the ultrasound imaging components 130 to the processing system 160 via the electrical conductors. In some embodiments, the same electrical conductors can be used for communication between the processing system 160 and the ultrasound imaging components 130.

The ultrasound device 110 includes an interface 156 at the proximal portion 112 of the flexible elongate member 116. In some embodiments, the interface 156 can include a handle. For example, handle can include one or more actuation mechanisms to control movement of the device 110, such as deflection of the distal portion 114. In some embodiments, the interface 156 can include a telescoping mechanism that allows for pullback of the device 110 through the lumen. In some embodiments, the interface 156 can include a rotation mechanism to rotate one or more components of the device 110 (*e*.*g*., the flexible elongate member 116 and the ultrasound imaging components 130). In some embodiments, the interface 156 includes a user interface component (*e*.*g*., one or more buttons, a switch, etc.) for a medical professional to selectively activate the ultrasound imaging components 130. In other embodiments, a user interface component of the PIM 150, the processing system 160 and/or the monitor 170 allows a medical profession to selectively activate the ultrasound imaging components 130. A conduit including, *e*.*g*., electrical conductors, extends between the interface 156 and the connector 108. The connector 108 can be configured to mechanically and/or electrically couple the device 110 to the PIM 150.

The ultrasound processing system 160, the PIM 150, and/or the intravascular ultrasound device 110 (*e*.*g*., the interface 156, the ultrasound imaging components 130, etc.) can include one or more controllers. The controllers can be integrated circuits, such as application specific integrated circuits (ASIC), in some embodiments. The controllers can be configured to select the particular transducer element(s) to be used for transmit and/or receive, to provide the transmit trigger signals to activate the transmitter circuitry to generate an electrical pulse to excite the selected transducer element(s), and/or to accept amplified echo signals received from the selected transducer element(s) via amplifiers of controllers. Multiple ASIC configurations with various numbers of master circuits and slave circuits can be used to create a single ultrasound wave or multi-firing ultrasound wave device.

In some embodiments, the PIM 150 performs preliminary processing of the ultrasound echo data prior to relaying the data to the console or processing system 160. In examples of such embodiments, the PIM 150 performs amplification, filtering, and/or aggregating of the data. In an embodiment, the PIM 150 also supplies high- and low-voltage DC power to support operation of the device 110 including circuitry associated with the ultrasound transducers 130. The PIM 150 can be an isolation device as, in various surgical settings, patient safety requirements mandate physical and electrical isolation of the patient from one or more high voltage components.

The ultrasound processing system 160 receives imaging data (*e*.*g*., electrical signals representative of the ultrasound echo data) from the ultrasound imaging components 130 by way of the PIM 150. The processing system 160 can include processing circuit, such as processor and/or memory. The ultrasound processing system 160 processes the data to reconstruct an image of the anatomy. The processing system 160 outputs image data such that an image of the anatomy 102, such as a cross-sectional IVUS image of a vessel, is displayed on the monitor 170. The processing system 160 and/or the monitor 170 can include one or more user interface elements (*e*.*g*., touchscreen, keyboard, mouse, virtual buttons on a graphical user interface, physical buttons, etc.) to allow a medical professional to control the device 110, including one or more parameters of the ultrasound imaging components 130.

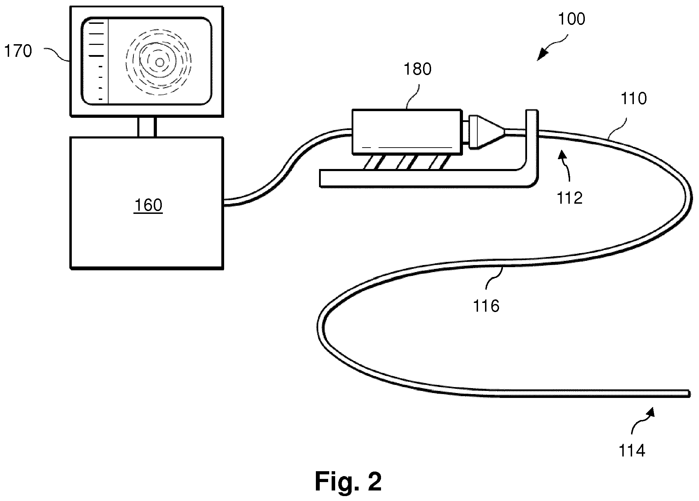

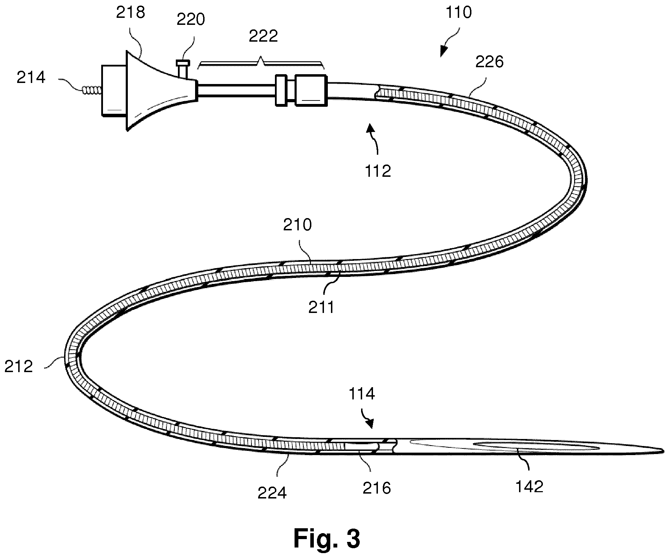

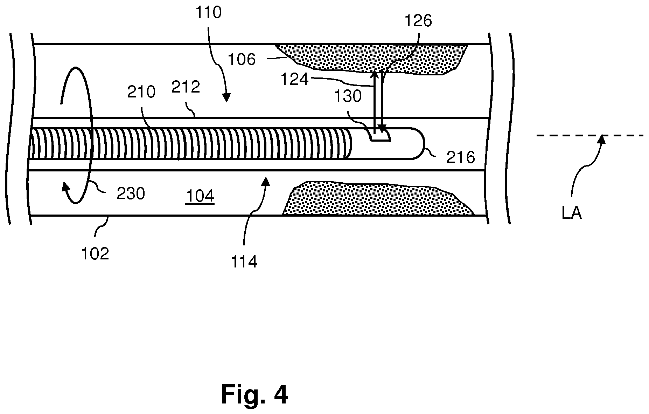

Fig. 2 is a diagrammatic, schematic view of the ultrasound system 100, according to an embodiment of the present disclosure. Fig. 3 is a diagrammatic, partial cutaway perspective view of the ultrasound device 110 according to an embodiment of the present disclosure. Fig. 4 is a diagrammatic view of the ultrasound device 110 in situ within the anatomy 102.

Referring in particular to Fig. 2, in some embodiments of the present disclosure, the ultrasound system 100 is a rotational IVUS imaging and therapeutic ultrasound system. The rotational ultrasound system 100 can include the ultrasound device 110, the console or processing system 160, and the monitor 170. As discussed in greater detail herein, the ultrasound device 110 includes ultrasound transducers 130 for imaging. The ultrasound device 110 can also include circuitry associated with the ultrasound transducers 130 mounted near the distal tip of the catheter, an electrical cable with one, two, three, four or more conductors, and the appropriate connector at the proximal portion 112 to support mechanical and/or electrical interconnection at a rotational interface. The body of the ultrasound device 110 can be referenced as the flexible elongate member 116. The distal portion 114 of the ultrasound device 110 is positioned within the anatomy 102 of the patient. The proximal portion 112 of the ultrasound device 110 is mechanically and/or electrically coupled to a movement device 180 of the system 100. The movement device includes one or more motors, associated circuitry, and/or other suitable components structurally arranged to impart rotational and/or longitudinal movement to one or more components of the ultrasound device 110, such as a drive cable 211. The movement device 180 can be referenced as a pullback device and/or a sled in some instances.

In some embodiments, the movement device 180 and the PIM 150 can be combined in single device. In other embodiments, the system 100 includes a PIM 150 distinct from the movement device 180. The PIM 150 generates the required sequence of transmit trigger signals and control waveforms to regulate the operation of the circuitry associated with the ultrasound imaging components 130, and processes the amplified echo signals received over the conductors of the electrical cable. The PIM 150 also supplies the DC high- and low-voltages to support operation of the ultrasound imaging components 130. In that regard, the PIM 150 is structurally arranged to supply DC voltages to the circuitry of the ultrasound device 110 across a rotational interface, using slip rings and/or the implementation of the active spinner technology described in U.S. Patent No. 8,403,856, which is hereby incorporated by reference in its entirety. In some embodiments, the PIM 150 supplies AC voltage to the ultrasound imaging components 130 using, *e*.*g*., a rotary transformer.

Figs. 3 and 4 illustrate additional detail regarding the structure of the rotational ultrasound device 110. Fig. 4 also shows the ultrasound device 110 in situ in the anatomy 102. In some respects, the ultrasound device 110 is similar to rotational IVUS catheters such as the Revolution^{®} catheter available from Volcano Corporation and described in U.S. Patent No. 8,104,479, or those disclosed in U.S. Patent Nos. 5,243,988 and 5,546,948. In that regard, the ultrasound device 110 includes an imaging core 210 and an outer catheter/sheath assembly 212. The imaging core 210 includes a flexible drive cable or shaft that is terminated at the proximal end of the proximal portion 112 by a rotational interface 214 providing electrical and mechanical coupling to the PIM 150. The imaging core 210 can also include one, two, three, four, or more electrical conductors in communication with the ultrasound imaging components 130. The distal portion 114 of the flexible drive shaft of the imaging core 210 is mechanically coupled to a proximal portion of a transducer housing 216 containing the ultrasound imaging components 130, and associated circuitry, as described in more detail herein.

The catheter/sheath assembly 212 includes a hub 218 that supports the rotational interface 214 and provides a bearing surface and a fluid seal between the rotating and nonrotating elements of the ultrasound device 110. The hub 218 includes a luer lock flush port 220 through which saline is injected to flush out the air within the sheath 212 and fill the inner lumen of the sheath 212 with an ultrasound-compatible fluid at the time of use of the ultrasound device 110. The saline or other similar flush is typically required since air does not readily conduct ultrasound. Saline also provides a biocompatible lubricant for the rotating drive cable of the imaging core 210. The hub 218 is coupled to a telescope 222 that includes nested tubular elements and a sliding fluid seal that permit the catheter/sheath assembly 212 to be lengthened or shortened to facilitate axial or longitudinal movement of the transducer housing within an acoustically transparent window 224 of the distal portion of the ultrasound device 110. In some embodiments, the window 224 is composed of thin-walled plastic tubing fabricated from material(s) that readily conduct ultrasound waves between the transducer and the vessel tissue with minimal attenuation, reflection, or refraction. A proximal shaft 226 of the catheter/sheath assembly 212 bridges the segment between the telescope 222 and the window 224, and is composed of a material or composite that provides a lubricious internal lumen and optimum stiffness, but without the need to conduct ultrasound. The guidewire entry/exit port 142 is provided at the distal portion of ultrasound device 110 in the illustrated embodiment.

The movement device 180 rotates the drive cable 211 of the imaging core 210 (*e*.*g*., in a clockwise or counter-clockwise direction 230) inside the polymer/plastic sheath 212 inserted into lumen 104 of the anatomy 102. Rotation of the drive cable 211 causes corresponding rotation of the housing 216, which is mechanically coupled to the drive cable. The ultrasound imaging components 130 are fixedly secured to the housing 216, and correspondingly rotate with the drive cable 211. The ultrasound imaging components 130 are oriented such that the respective ultrasound beams 124 propagate generally perpendicular to the longitudinal axis LA of the ultrasound device 110. The fluid-filled sheath 212 protects the tissue of the anatomy from the spinning ultrasound imaging components 130 and the drive cable 211 while permitting ultrasound signals to freely propagate. As the driveshaft rotates (*e*.*g*., at 30 revolutions per second), the ultrasound imaging components 130 are selectively and/or periodically excited with a high voltage pulse to emit a burst of ultrasound energy. The ultrasound imaging components 130 listen for the returning ultrasound echoes 126 reflected from various tissue structures of the anatomy 102, such as the occlusion 106. Based on the IVUS imaging data obtained by the ultrasound imaging components 130, the IVUS imaging system 160 assembles a two-dimensional image of the vessel cross-section from a sequence of several hundred of these ultrasound pulse/echo acquisition sequences occurring during a single revolution of the ultrasound imaging components 130.

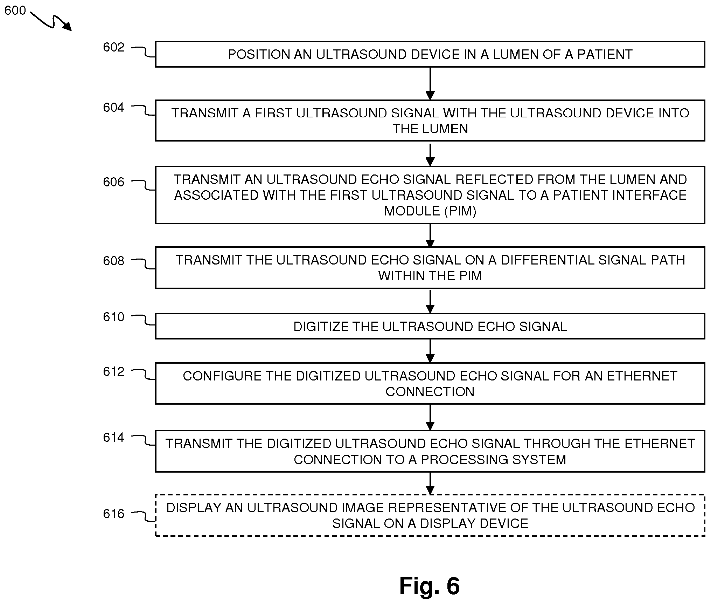

The ultrasound imaging components 130 may be mechanically coupled to the housing 216 using any suitable attachment mechanism, such as adhesive, welding, soldering, etc. The ultrasound imaging components 130 may be positioned adjacent to one another along the longitudinal axis LA. In some instances, the ultrasound imaging components 130 can be referenced as being aligned in series. In some embodiments, the ultrasound imaging components 130 are positioned side-by-side along an axis perpendicular to the longitudinal axis LA. In some embodiments, the ultrasound imaging components 130 are disposed on opposite sides of the housing 216. For example, in the orientation of the housing 216 illustrated in Fig. 6, one of the ultrasound imaging components 130 can be disposed on one side of the housing 216 (*e*.*g*., facing up), while another of the ultrasound imaging components 130 can be disposed on the opposing side of the housing 216 (*e*.*g*., facing down). For example, the ultrasound imaging components 130 can be configured to emit ultrasound energy in opposite directions. In the illustrated embodiment, the ultrasound imaging components 130 are individual ultrasound elements. In other embodiments, the ultrasound imaging components 130 can be a one or two dimensional ultrasound array including two or more ultrasound transducers.

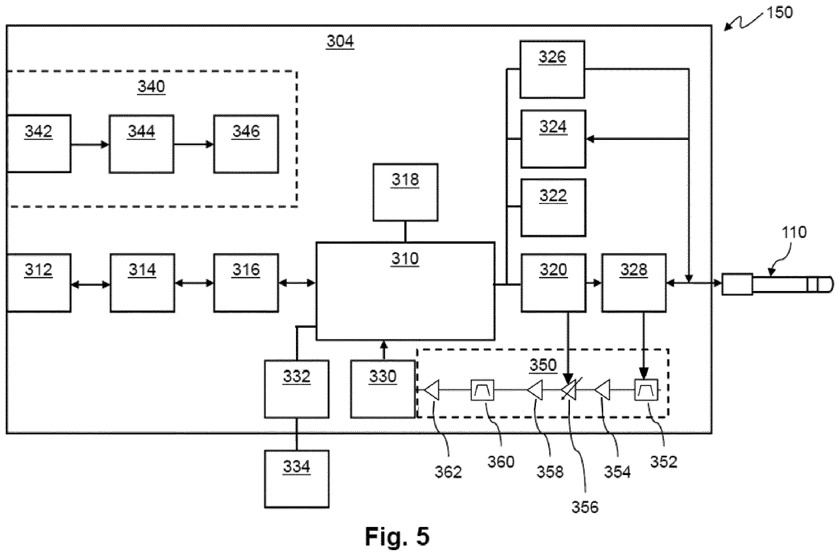

Fig. 5 is a diagrammatic schematic view of a PIM 150. In some embodiments, the PIM 150 is communicatively disposed between the ultrasound device 110 and the processing system 160. The PIM 150 may be used to transmit commands and signals to the ultrasound device 110, as well as to receive, process, and transmit ultrasound echo signals from the ultrasound device 110. In some embodiments, these ultrasound echo signals are transmitted along a differential signal path in the PIM 150, and are digitized and formatted for Ethernet transmission to the ultrasound processing system 160.

The PIM 150 may include an outer housing 304. The housing 304 may be suitable for use in a sterile environment (*i*.*e*., water resistant) and may be sized to be suitable for use on an operating table. In some embodiments, the housing 304 includes internal sections to house various components. For example, the housing 304 may include particular housing sections to contain the power system 340, the signal chain 350, and the controller 310 and associated components.

The controller 310 of the PIM 150 may be configured to transmit signals to other elements of the PIM 150 as well as to external devices, such as the ultrasound device 110, processing system 160, and monitor 170. In some embodiments, the controller 310 is a field-programmable gate array (FPGA). In other embodiments, the controller 310 is a central processing unit (CPU), a digital signal processor (DSP), an application-specific integrated circuit (ASIC), another hardware device, a firmware device, or any combination thereof configured to perform the operations described herein with reference to the controller 310 as shown in Fig. 5 above.

The controller 310 may be connected to a memory 318. In some embodiments, the memory is a random access memory (RAM). In other embodiments, the memory 318 is a cache memory (*e*.*g*., a cache memory of the controller 310), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In some embodiments, the memory 318 may include a non-transitory computer-readable medium. The memory 318 may store instructions. The instructions may include instructions that, when executed by a processor, cause the processor to perform operations described herein with reference to the controller 310 in connection with embodiments of the present disclosure.

The controller 310 may be connected to a catheter motor 326, EEPROM 324, transmitter 322, and a time gain compensation (TGC) control 320. In some embodiments, the catheter motor 326 is configured to move the ultrasound device 110 within a lumen. The catheter motor 326 may include a rotational component for rotating a portion of the ultrasound device 110. The catheter motor 326 may also include a motor for moving the ultrasound device 110 along lumens within the body of the patient.

The transmitter 322 may be any type of transmission device for sending signals to the ultrasound device 110. In some embodiments, the controller 310 is configured to control the ultrasound device 110 by sending signals through the transmitter 322. In this way, the controller 310 may be configured to drive the transmission of ultrasound signals by the ultrasound device 110. The direction of transmission and signal strength of the ultrasound signals may be controlled by the controller 310. The transmitter 322 may be connected to a transmit/receive (T/R) switch 328. In some embodiments, the T/R switch 328 may be configured to change between transmit and receive modes. For example, the controller 310 may send a signal to the ultrasound device 110 while the T/R switch 328 is in transmit mode. Data (such as ultrasound echo signals) may be transmitted back from the ultrasound device 110 to the PIM 150. This data may be stored by the EEPROM 324. When the ultrasound echo signals are transmitted back from the ultrasound device 110 to the PIM 150, the T/R switch 328 may be set to receive mode to receive and direct the ultrasound echo signals along the correct signal route.

The ultrasound echo signals may be received by the PIM 150 and directed along a differential signal route. In some embodiments, the differential signal route may include a signal chain 350 including one or more elements 352, 354, 356, 358, 360, 362. The differential signal route may help to cancel common mode noise, and in particular "white noise/flicker" which can occur in existing image processing systems. The differential signal route and associated signal chain 350 may result in a more noise free signal and improved image quality. The signal chain 350 may provide filtering and programmable gain functions. In some embodiments, the TGC control 320 is a time varying gain that adjusts for signal loss as the distance between the PIM 150 and the ultrasound device 110 increases. The gain is typically reduced for near reflections and gradually increased for distant reflections. The amount of gain over distance may be controlled, for example by the controller 310 of the PIM 150. In some embodiments, the TGC control may be configured to control the signal amplification of the received ultrasound echo signals. The TGC control 320 may also be configured to set the receive path for the ultrasound echo signals along the signal chain 350. The signal chain 350 may include bandpass filters 352, 360 and amplifiers 354, 356, 358, 362. For example, the ultrasound echo signals from the ultrasound device 110 may be passed in order through a first bandpass filter 352, a first fixed amplifier 354, a variable gain amplifier 356, a first buffer amplifier 358, a second bandpass filter 360, and a second buffer amplifier 362. In some embodiments, the bandpass filters 352, 360 allow signals between 20 and 40 MHz. In other embodiments, the bandpass filters allow other ranges of signals, such as 10 to 50 MHz, 5 to 60 MHz, and other ranges.

After the signals are passed through the signal chain 350, they may be transmitted to an analog to digital converter (ADC) 330. The ADC 330 may digitize the ultrasound echo signals for processing by the controller 310. The signals may then be prepared for transmission to the ultrasound processing system 160. In some embodiments, the signals from the ultrasound device 110 may be transmitted by an Ethernet connection to the ultrasound processing system 160. In this case, the signals from the ultrasound device 110 (which have been digitized by the ADC 330) are transmitted to an Ethernet physical layer (PHY) 316. The Ethernet PHY may be configured to convert the signals from the ultrasound device 110 for an Ethernet connection. The converted signals may then be passed to an isolation transformer 314. In some embodiments, the isolation transformer 314 meets IEC-60601 requirements for Ethernet-based transformers. The signals are then passed to the Ethernet connection 312 for transmission to the ultrasound processing system 160. The Ethernet connection 312 may include one or more Ethernet cables as well as associated ports.

In other embodiments, the PIM 150 may be configured to transmit data from the ultrasound device 110 to the ultrasound processing system 160 via standards other than Ethernet, such as USB (and in particular, USB 3.0). In this case, the PIM 150 may include a USB connector and the signals from the ultrasound device 110 may be configured for use with USB.

The PIM 150 may include a pullback motor 332 which may be used to pull the ultrasound device 110 through a lumen to collect imaging data. The pullback motor 332 may be configured to pull the ultrasound device 110 at a constant speed. The pullback motor 332 may be connected to an external pullback sled 334.

The PIM 150 may include a power system 340. In some embodiments, the PIM 150 is powered by power over Ethernet (PoE). In this case, power may be input through the Ethernet connection 312 or through another Ethernet connection on the PIM 150. In other embodiments, the PIM 150 is powered by a power input 342 within the power system 340. The power input 342 may be AC/DC power. The power input 342 may be connected to an isolation power module 344, which may convert the power to DC/DC. The power may then be distributed throughout the PIM 150 by the power distributor 346.

Fig. 6 provides a flow diagram illustrating a method 600 of intravascular ultrasound imaging. As illustrated, the method 600 includes a number of enumerated steps, but embodiments of the method 600 may include additional steps before, after, and in between the enumerated steps. In some embodiments, one or more of the enumerated steps may be omitted, performed in a different order, or performed concurrently. The method 600 may be performed using any of the systems and devices referred to in Figs. 1-5.

At step 602, the method 600 may include positioning an ultrasound device in a body lumen of a patient. The ultrasound device may be similar to the ultrasound device 110 as shown in Figs. 1, 4, and 5. In particular, the ultrasound device may be an intravascular rotational ultrasound device with one or more imaging ultrasound transducer elements at the distal portion of a rotating drive cable. The step 602 can include placing a sheath and the imaging core/drive cable within the lumen of the anatomy. The drive cable can be disposed within the sheath of the ultrasound device.

At step 604, the method 600 may include transmitting a first ultrasound signal with the ultrasound device into the lumen. The first ultrasound signal may be transmitted with one or more ultrasound elements of the ultrasound device. In some embodiments, the transmission of the first ultrasound signal may be controlled by a patient interface module (PIM) such as PIM 150 as shown in Figs. 1 and 5. For example, a controller of the PIM may be used to send a signal to the ultrasound device, which may in turn be transmitted into the lumen by the one or more ultrasound elements of the ultrasound device. Step 604 may be performed while the drive cable of the ultrasound device and the one or more ultrasound elements are rotating within the sheath positioned inside the lumen. In that regard, the method 600 can include connecting the ultrasound device and/or the drive cable to a movement device, such as a pullback device, that is configured to rotate and/or longitudinally translate the ultrasound device. The first ultrasound signal may be reflected off anatomy (*e*.*g*., tissue, blood vessel, plaque, etc.) within the lumen in the form of ultrasound echoes, some of which may travel back toward the first ultrasound element. These ultrasound echo signals may be received by the ultrasound device, such as with one or more transducer elements.

At step 606, an ultrasound echo signal associated with the first ultrasound signal may be transmitted to the PIM. In some embodiments, the ultrasound echo signal is received by a transmit/receive (T/R) switch, such as T/R switch 328 as shown in Fig. 5. The ultrasound echo signal may be processed by the PIM in preparation for its use in creating ultrasound images of the lumen.

At step 608, the ultrasound echo signal may be transmitted on a differential signal path within the PIM. In some embodiments, the differential signal path may help to reduce noise. The differential signal path may include a signal chain with one or more amplifiers and buffers. In some embodiments, the differential signal path includes, in order, a first bandpass filter, a first fixed amplifier, a variable gain amplifier, a first buffer amplifier, a second bandpass filter, and a second buffer amplifier. In other embodiments, the differential signal path includes other combinations of elements.

At step 610, the ultrasound echo signal may be digitized. In some embodiments, after passing along the differential signal path, the ultrasound echo signal is transmitted to an ADC within the PIM. The ADC may be used to digitize the ultrasound echo signal. The digitized ultrasound echo signal may then be transmitted to a controller within the PIM.

At step 612, the digitized ultrasound echo signal may be configured for an Ethernet connection. In some embodiments, this includes transmitting the digitized ultrasound echo signal to an Ethernet physical layer (PHY) with the controller of the PIM. Step 612 may also include passing the ultrasound echo signal through an isolation transformer and to an Ethernet connector.

At step 614, the digitized ultrasound signal may be transmitted through the Ethernet connection to a processing system. The processing system may be the processing system 160 as shown in Fig. 1. Step 614 may be carried out by using one or more Ethernet cables connected between the PIM and the processing system. The processing system may be used to further process the digitized ultrasound echo signal to produce ultrasound images of the lumen of the patient.

At step 616, an ultrasound image representative of the ultrasound echo signal may optionally be displayed on a display device. The display device may be similar to the monitor 170 as shown in Fig. 1. For example, the image can be an IVUS image of a blood vessel.

## Claims

1. An intraluminal ultrasound imaging system (100) comprising:
a patient interface module (PIM) (150) communicatively disposed between a processing system (160) and an intraluminal ultrasound device (110) configured to be positioned within a body lumen of a patient, the PIM (150) comprising a transmitter, an analog to digital converter (ADC) (330), and a communication device comprising a communication cable, wherein the PIM (150) is configured to:
transmit, with the transmitter, a first signal to the intraluminal ultrasound device (110);
receive an ultrasound echo signal associated with the first signal from the intraluminal ultrasound device (110);
digitize the ultrasound echo signal with the ADC (330);
convert the digitized ultrasound echo signal to a second signal communicable via the communication device; and
transmit the second signal to the processing system (160) via the communication cable of the communication device,
**characterised in that** the PIM (150) is configured to transmit the ultrasound echo signal along differential signal path of the PIM (150) to the ADC (330), wherein said differential signal path is configured to cancel common mode noise.

2. The intraluminal ultrasound imaging system of claim 1, wherein the processing system (160) is configured to generate an intraluminal ultrasound image representative of the ultrasound echo signal based on the second signal and to display the intraluminal ultrasound image on a display device in communication with the processing system.

3. The intraluminal ultrasound imaging system of claim 1, wherein
the communication cable is an Ethernet cable, or
the communication cable is a USB cable.

4. The intraluminal ultrasound imaging system of claim 1, wherein the PIM (150) further
comprises a controller in communication with the transmitter, the ADC, and the communication device.

5. The intraluminal ultrasound imaging system of claim 4, wherein the controller is a field-programmable gate array (FPGA).

6. The intraluminal ultrasound imaging system of claim 1, wherein the intraluminal ultrasound device (110) comprises:
a rotatable, flexible elongate drive cable comprising a proximal portion and a distal portion; and
an ultrasound element disposed at the distal portion of the drive cable and configured to obtain imaging data of the body lumen while rotating.

7. The intraluminal ultrasound imaging system of claim 1, wherein the intraluminal ultrasound device is an intravascular ultrasound (IVUS) device configured to be positioned within a blood vessel.

8. The intraluminal ultrasound imaging system of claim 1, wherein the differential signal path comprises one or more amplifiers and bandpass filters.

9. A method of intraluminal ultrasound imaging, comprising:
transmitting (604) a first signal with an intraluminal ultrasound device (110) positioned within a body lumen of a patient;
receiving, with a patient interface module (PIM) (150) communicatively disposed between the intraluminal ultrasound device and a processing system (160), an ultrasound echo signal associated with the first signal from the intraluminal ultrasound device;
transmitting (608), with the PIM (150), the ultrasound echo signal along a differential signal path to an analog to digital converter (ADC) (330) within the PIM (150) in order to cancel common mode noise;
digitizing (610) the ultrasound echo signal with the ADC (330) within the PIM;
converting the digitized ultrasound echo signal to a second signal communicable via a communication device; and
transmitting (614) the second signal to the processing system via the communication device.

10. The method of claim 9, further comprising displaying (616), with a display device in communication with the processing system, an intraluminal ultrasound image representative of the ultrasound echo signal.

11. The method of claim 10, further comprising formatting, by the PIM (150), the ultrasound echo signal according to an image display format of the display device.

12. The method of claim 9, wherein
the communication device is an Ethernet cable, or
the communication device is a USB cable.

13. The method of claim 9, wherein a controller of the PIM (150) is a field-programmable gate array (FPGA).

14. The method of claim 9, further comprising converting the digitized ultrasound echo signal to a second signal with an Ethernet physical layer (PHY) device.

15. The method of claim 9, wherein the intraluminal ultrasound device (110) comprises:
a rotatable, flexible elongate drive cable comprising a proximal portion and a distal portion; and
an ultrasound element disposed at the distal portion of the drive cable and configured to obtain imaging data of the body lumen while rotating.

## Patentansprüche

1. Ein intraluminales Ultraschallbildgebungssystem (100), das Folgendes umfasst:
ein Patientenschnittstellenmodul (PIM) (150), das kommunikativ zwischen einem Verarbeitungssystem (160) und einem intraluminalen Ultraschallgerät (110) angeordnet ist, das im Körperlumen eines Patienten positioniert werden kann, wobei die PIM (150) einen Sender, einen Analog-Digital-Wandler (ADC) (330) und ein Kommunikationsgerät mit einem Kommunikationskabel umfasst, wobei die PIM (150) folgende Schritte durchführt:
mit dem Sender Übertragen eines ersten Signals an das intraluminale Ultraschallgerät (110);
Abrufen eines Ultraschallechosignals vom intraluminalen Ultraschallgerät (110), das dem ersten Signal zugeordnet ist;
Digitalisieren des Ultraschallechosignals mit dem ADC (330) ;
Umwandeln des digitalisierten Ultraschallechosignal in ein zweites Signal, das mit dem Kommunikationsgerät übertragen werden kann; und
Übertragen des zweiten Signals an das Verarbeitungssystem (160) über das Kommunikationskabel des Geräts, **dadurch gekennzeichnet, dass** die PIM (150) das Ultraschallechosignal entlang eines Differenzsignalpfads der PIM (150) an den ADC (330) überträgt, wobei der Differenzsignalpfad so konfiguriert ist, dass er das Gleichtaktrauschen unterdrückt.

2. Das intraluminale Ultraschall-Bildgebungssystem gemäß Anspruch 1, wobei das Verarbeitungssystem (160) beruhend auf dem zweiten Signal ein dem Ultraschallechosignal entsprechendes intraluminales Ultraschallbild generiert und das intraluminale Ultraschallbild auf einem mit dem Verarbeitungssystem verbundenen Anzeigegerät anzeigt.

3. Das intraluminale Ultraschall-Bildgebungssystem gemäß Anspruch 1, wobei es sich beim Kommunikationskabel um ein Ethernet-Kabel oder um ein USB-Kabel handelt.

4. Das intraluminale Ultraschall-Bildgebungssystem gemäß Anspruch 1, wobei das PIM (150) zudem eine Steuerung umfasst, die mit dem Sender, dem ADC und dem Kommunikationsgerät kommuniziert.

5. Das intraluminale Ultraschall-Bildgebungssystem gemäß Anspruch 4, wobei es sich bei der Steuerung um ein Field Programmable Gate Array (FPGA) handelt.

6. Das intraluminale Ultraschall-Bildgebungssystem gemäß Anspruch 1, wobei das intraluminale Ultraschallgerät (110) Folgendes umfasst:
ein drehbares, flexibles, langes Antriebskabel mit einem proximalen und einem distalen Abschnitt; und
ein Ultraschallelement, das am distalen Abschnitt des Antriebskabels angeordnet ist und beim Drehen Bildgebungsdaten für das Körperlumen erfasst.

7. Das intraluminale Ultraschall-Bildgebungssystem gemäß Anspruch 1, wobei es sich beim intraluminalen Ultraschallgerät um ein intravaskuläres Ultraschallgerät (IVUS) handelt, das in einem Blutgefäß positioniert wird.

8. Das intraluminale Ultraschall-Bildgebungssystem gemäß Anspruch 1, wobei der Differenzsignalpfad mindestens einen Verstärker und Bandfilter umfasst.

9. Eine Methode für die intraluminalen Ultraschallbildgebung, die folgende Schritte umfasst:
Übertragen (604) eines ersten Signals mit einem intraluminalen Ultraschallgerät (110), das in einem Körperlumen eines Patienten positioniert ist;
mit einem Patientenschnittstellenmodul (PIM) (150), das kommunikativ zwischen dem intraluminalen Ultraschallgerät und einem Verarbeitungssystem (160) angeordnet ist, Abrufen eines Ultraschallechosignals, das dem ersten Signal vom intraluminalen Ultraschallgerät zugeordnet ist;
Übertragen (608) des Ultraschallechosignals mit der PIM (150) entlang eines Differenzsignalpfads zu einem Analog-Digital-Wandler (ADC) (330) innerhalb der PIM (150), um das Gleichtaktrauschen zu unterdrücken;
Digitalisieren (610) des Ultraschallechosignals mit dem ADC (330) der PIM;
Umwandeln des digitalisierten Ultraschallechosignal in ein zweites Signal, das mit dem Kommunikationsgerät übertragen werden kann; und
Übertragen (614) des zweiten Signals an das Verarbeitungssystem mit dem Kommunikationsgerät.

10. Die Methode gemäß Anspruch 9, die zudem das Anzeigen (616) eines dem Ultraschallechosignal entsprechenden intraluminalen Ultraschallbilds mit einem Anzeigegerät umfasst, das mit dem Verarbeitungssystem kommuniziert.

11. Die Methode gemäß Anspruch 10, die zudem das Formatieren des Ultraschallechosignals gemäß des Bildanzeigeformats des Anzeigegeräts mit der PIM (150) umfasst.

12. Die Methode gemäß Anspruch 9, wobei es sich beim Kommunikationsgerät um ein Ethernet-Kabel oder
um ein USB-Kabel handelt.

13. Die Methode gemäß Anspruch 9, wobei es sich bei der Steuerung der PIM (150) um ein Field Programmable Gate Array (FPGA) handelt.

14. Die Methode gemäß Anspruch 9, die zudem das Umwandeln des digitalisierten Ultraschallechosignals in ein zweites Signal mit einem Ethernet-Gerät der physikalischen Schicht (PHY) umfasst.

15. Die Methode gemäß Anspruch 9, wobei das intraluminale Ultraschallgerät (110) Folgendes umfasst:
ein drehbares, flexibles, langes Antriebskabel mit einem proximalen und einem distalen Abschnitt; und
ein Ultraschallelement, das am distalen Abschnitt des Antriebskabels angeordnet ist und beim Drehen Bildgebungsdaten für das Körperlumen erfasst.

## Revendications

1. Système d'imagerie intraluminale par ultrasons (100) comprenant :
un module d'interface patient (PIM) (150) disposé en communication entre un système de traitement (160) et
un dispositif intraluminal à ultrasons (110) conçu pour être positionné dans une lumière corporelle d'un patient, le PIM (150) comprenant un émetteur, un convertisseur analogique-numérique (ADC) (330), et un dispositif de communication comprenant un câble de communication, dans lequel le PIM (150) est conçu :
pour transmettre, à l'aide de l'émetteur, un premier signal au dispositif intraluminal à ultrasons (110) ;
pour recevoir un signal d'écho ultrasonore associé au premier signal provenant du dispositif intraluminal à ultrasons (110) ;
pour numériser le signal d'écho ultrasonore à l'aide du ADC (330) ;
pour convertir le signal d'écho ultrasonore numérisé en un second signal communicable par l'intermédiaire du dispositif de communication ; et
pour transmettre le second signal au système de traitement (160) par l'intermédiaire du câble de communication du dispositif de communication,
**caractérisé en ce que** le PIM (150) est conçu pour transmettre le signal d'écho ultrasonore le long d'un chemin de signal différentiel du PIM (150) à l'ADC (330), dans lequel ledit chemin de signal différentiel est configuré pour supprimer le bruit de mode commun.

2. Système d'imagerie intraluminale par ultrasons selon la revendication 1, dans lequel le système de traitement (160) est conçu pour générer une image intraluminale par ultrasons représentant l'écho ultrasonore en fonction du second signal et pour afficher l'image intraluminale par ultrasons sur un dispositif d'affichage en communication avec le système de traitement.

3. Système d'imagerie intraluminale par ultrasons selon la revendication 1, dans lequel
le câble de communication est un câble Ethernet ou
le câble de communication est un câble USB.

4. Système d'imagerie intraluminale par ultrasons selon la revendication 1, dans lequel le PIM (150) comprend en outre un dispositif de commande en communication avec l'émetteur, l'ADC et le dispositif de communication.

5. Système d'imagerie intraluminale par ultrasons selon la revendication 4, dans lequel le dispositif de commande est un réseau de portes programmables (FPGA).

6. Système d'imagerie intraluminale par ultrasons selon la revendication 1, dans lequel le dispositif intraluminal à ultrasons (110) comprend :
un câble d'entraînement allongé flexible et rotatif comprenant une partie proximale et une partie distale ; et
un élément ultrasonore disposé au niveau de la partie distale du câble d'entraînement et conçu pour obtenir des données d'imagerie de la lumière corporelle lors de la rotation.

7. Système d'imagerie intraluminale par ultrasons selon la revendication 1, dans lequel le dispositif intraluminal à ultrasons est un dispositif intravasculaire à ultrasons (IVUS) conçu pour être positionné à l'intérieur d'un vaisseau sanguin.

8. Système d'imagerie intraluminale par ultrasons selon la revendication 1, dans lequel le chemin de signal différentiel comprend au moins un amplificateur et un filtre passe-bande.

9. Procédé d'imagerie intraluminale par ultrasons, comprenant :
la transmission (604) d'un premier signal à l'aide d'un dispositif intraluminal à ultrasons (110) positionné dans une lumière corporelle d'un patient ;
la réception, à l'aide d'un module d'interface patient (PIM) (150) disposé en communication entre le dispositif intraluminal à ultrasons et le système de traitement (160),
un signal d'écho ultrasonore étant associé au premier signal provenant du dispositif intraluminal à ultrasons ;
la transmission (608), à l'aide du PIM (150), du signal d'écho ultrasonore le long d'un chemin de signal différentiel à un convertisseur analogique-numérique (ADC) (330) à l'intérieur du PIM (150) pour supprimer le bruit de mode commun ;
la numérisation (610) du signal d'écho ultrasonore à l'aide de l'ADC (330) dans le PIM ;
la conversion du signal d'écho ultrasonore numérisé en un second signal pouvant être communiqué par l'intermédiaire d'un dispositif de communication ; et
la transmission (614) du second signal au système de traitement par l'intermédiaire du dispositif de communication.

10. Procédé selon la revendication 9, comprenant en outre l'affichage (616), à l'aide d'un dispositif d'affichage en communication avec le système de traitement, d'une image intraluminale par ultrasons représentant le signal d'écho ultrasonore.

11. Procédé selon la revendication 10, comprenant en outre le formatage, à l'aide du PIM (150), du signal d'écho ultrasonore selon un format d'affichage d'image du dispositif d'affichage.

12. Procédé selon la revendication 9, dans lequel
le dispositif de communication est un câble Ethernet ou
le dispositif de communication est un câble USB.

13. Procédé selon la revendication 9, dans lequel un dispositif de commande du PIM (150) est un réseau de portes programmables (FPGA).

14. Procédé selon la revendication 9, comprenant en outre la conversion du signal d'écho ultrasonore numérisé en un second signal à l'aide d'un dispositif de couche physique Ethernet (PHY).

15. Procédé selon la revendication 9, dans lequel le dispositif intraluminal à ultrasons (110) comprend :
un câble d'entraînement allongé flexible et rotatif comprenant une partie proximale et une partie distale ; et
un élément ultrasonore disposé au niveau de la partie distale du câble d'entraînement et conçu pour obtenir des données d'imagerie de la lumière corporelle lors de la rotation.
